# EUROPEAN PATENT SPECIFICATION

(11) **EP 2 999 508 B1**
(45) Date of publication and mention of the grant of the patent: **15.03.2017**
(21) Application number: 14720698.1
(22) Date of filing: 24.03.2014
(51) Int. Cl.: A61M 16/00

(54) **EQUIPMENT FOR REHABILITATIVE RESPIRATORY PHYSIOTHERAPY**
VORRICHTUNG FÜR RESPIRATORISCHE REHABILITATIONSPHYSIOTHERAPIE
ÉQUIPEMENT POUR PHYSIOTHÉRAPIE RESPIRATOIRE DE RÉÉDUCATION

(30) Priority: 20.05.2013 IT BS20130072
(43) Date of publication of application: 30.03.2016
(73) Proprietor: FLAEM NUOVA S.p.A., 25010 Desenzano del Garda (BS) (IT)
(72) Inventor: ABATE, Riccardo, I-25010 Desenzano del Garda (BS) (IT); ALBERTELLI, Roberto, I-25010 Desenzano del Garda (BS) (IT)
(74) Representative: Pes, Matteo
(86) International application number: PCT/IB2014/060104
(87) International publication number: WO 2014/188286

(56) References cited:
- EP-A2- 2 186 538
- WO-A1-96/24407
- WO-A1-2010/038233
- US-A- 2 918 917
- US-B1- 6 581 596

## Description

### Field Of The Invention

The present invention relates to an equipment for rehabilitative respiratory physiotherapy and in particular to a non-invasive equipment for removing the tracheobronchial secretions.

### State of the art

In some parts of the human respiratory system, secretions can accumulate for different reasons.

For example, for some surgical operations involving the chest or the abdomen, a prolonged anaesthesia is required and the patient mobility is restricted for a period, affecting his/her psychophysical recovery. The restricted mobility of abdomen and chest reduces the expansion during respiration and thus the oxygen supply and, at the same time, causes an increase of the secretions. Often, particularly debilitated patients cannot effectively cough to remove the secretions.

Other times, the patients suffer from pathologies that cause an abnormal production of secretions. This happens for example with the cystic fibrosis.

The airway treatment for removing secretions, i.e. the rehabilitative respiratory physiotherapy, can be carried out by means of several commercially available equipments, usually interacting with the airways of the patient to modify the breathing out phase of the patient himself, and particularly to adjust the pressure or the flow rate of breathed out air.

The PEP mask (acronym for *Positive Expiratory Pressure*) is very common, especially for treating patients suffering from chronic bronchial obstruction. Substantially, the PEP mask can be positioned on the face to surround mouth and nose and comprises a one-way valve and an adjustable resistance intercepting the valve outlet of breathed out air. By breathing with the PEP mask, a positive endobronchial pressure can be obtained during the breathing out phase.

The positive pressure acts to protract the opening of the airways along the whole breathing out phase, preventing the bronchial collapse in areas with damaged and unstable walls. Therefore the transitory pressure increase facilitates the ventilation of the more peripheral lung areas, the re-expansion of poorly ventilated, or not ventilated at all, areas and the secretion mobilization from the peripheral areas to the centre of the bronchial tubes.

With the trade name "UNIKO-TPEP E" is commercially available an equipment using the operating principle of the PEP mask; in particular, the equipment generates a positive pressure in the breathing out phase lasting about two thirds of the phase itself and allows the patient to terminate breathing out in a spontaneous manner, i.e. at atmospheric pressure.

An equipment widely used even in hospitals is known as FLUTTER. Basically, it comprises a mouthpiece having a PEP function, but is further provided with a resistance than can vary over time in an oscillatory way. The resistance, countering the patient breathing out in a oscillatory way, causes the onset of an oscillatory-type positive expiratory pressure, ranging from 10 to 20 cmH₂O in the airways, such as to facilitate the mucus detachment from the bronchial walls. The positive expiratory pressure varies slowly, i.e. with a frequency of no more than 15 Hz.

The Italian Patent Application MI2008A001315 relates to an equipment comprising an air compressor connected to a facial mask and arranged such as to accelerate the air the patient breathes out, that is to increase the instantaneous air flow rate in the breathing out phase, with a view to create a depression in the airways such as to facilitate the secretion detachment. An equipment exploiting this technical solution is known under the trade name "FREE ASPIRE".

Equipments applying pressure waves to the air insufflated and exsufflated to/from the patient are used in hospitals, but such equipments are mostly used in assisted ventilation, in other words they provide or draw air to/from the patient which otherwise would not be able to breathe autonomously.

At rest, a healthy person performs/makes about 15 breathing cycles per minute, where the term "*breathing cycle"* means breathing in followed by breathing out. Usually, at rest, a healthy person sucks on about 500 cm³ air in 2 seconds.

Therefore, in case of people suffering from pulmonary failure, the equipments have to compensate for the loss of activity of the patient by insufflating to him air volumes of at least 500 cm³ in 2 seconds, but without overdoing to avoid barotraumas.

The Patent Application US 2001/0007256 describes a device for mechanical ventilation, evidently intended to be used in hospital. The device comprises an endotracheal cannula to be inserted in patient trachea, a unit for supplying air, possibly containing pure oxygen as additive, and a membrane element operating to apply an oscillatory pressure to the air supplied to the patient. Further, the device comprises a discharge of the carbon dioxide the patient breathe produces and a pressure gauge. The membrane element transmits pressure waves to the air flow supplied to the patient. The apparatus is fully adjustable, that is to say the operator, or doctor, can adjust the frequency and the amplitude of pressure oscillations, and the air flow rate supplied to the patient. A controller keeps the air flow rate constant upon changes in amplitude and frequency of the pressure oscillations set by the doctor. In this way the level of assisted ventilation of the patient is maintained at an adequate level.

The operation provides that pressure waves are applied to the air flow the patient breathes in as well as to the air flow he/she breathes out (par. 22). The described maximum value of the frequency of the pressure waves applied to the air breathed in by the patient is 15 Hz (par. 91, par. 101, par. 102).

EP 1106197 also describes a device for the pulmonary assisted ventilation provided with an endotracheal cannula for supplying air to the patient. The device comprises sensors able to detect any possible attempt of the patient to breathe spontaneously. Signals generated by the sensors are used by a regulating unit to feedback regulate the flow rate and/or the pressure of the air flow supplied to the patient, such as to compensate for its pulmonary failure (col. 3, lines 45-52, col. 4, lines 5-15). The device comprises an oscillator operating to apply pressure waves to the breathed in and out air flow. Amplitude and frequency of waves can be adjusted. The frequency is about 3 Hz (par. 9).

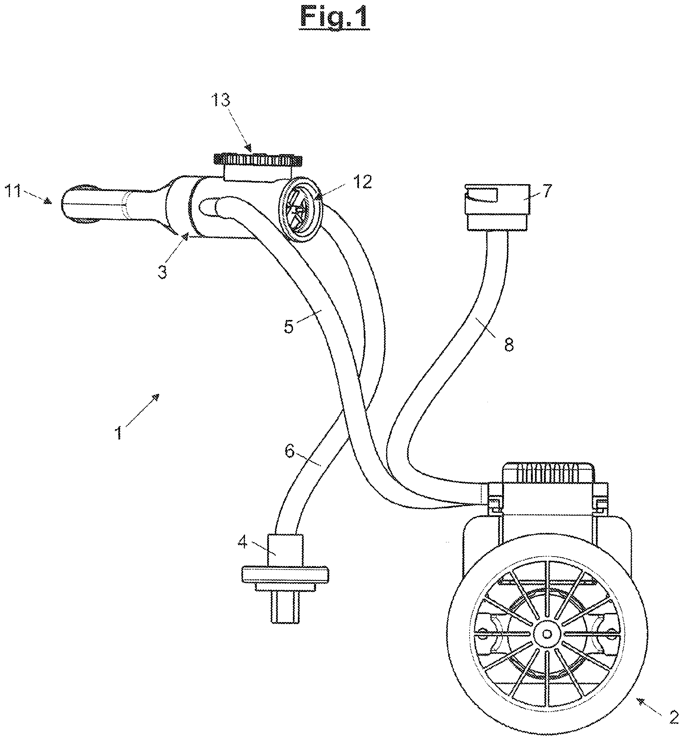

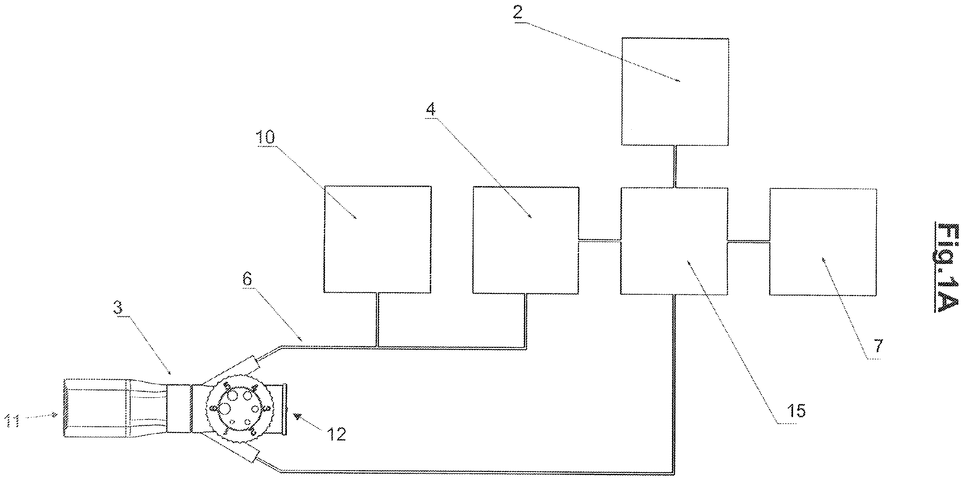

US 2005/0039749 describes a CPAP device provided with a sensor detecting the beginning of a breathing-in phase of the patient (n. 26 in fig. 1). To assist the patient, when he/she breathes in, the device applies only a positive pressure (par. 22). The pressure is provided by a blower (n. 30 in fig. 1).

US 2918917 describes an apparatus for treating a patient by vibrating a column of gas which is in communication with his airway, the column being vibrated at a rate which is greater than the patient's normal rate of inhalation and exhalation.

The Applicant found that available solutions based on PEP, FLUTTER systems and the like, can be improved, and solutions mostly used in hospitals for the pulmonary ventilation of not self-sufficient patients are bulky, difficult to transport and should be used by medical staff.

### Summary of the invention

It is therefore an object of the present invention to provide an equipment for rehabilitative respiratory physiotherapy, and in particular for pulmonary physiotherapy, allowing to effectively remove the secretions from the airways of self-sufficient patients and without necessarily requiring the intervention of medical staff.

It is a further object of the present invention to provide an equipment for the rehabilitative respiratory physiotherapy, and in particular for the pulmonary physiotherapy, which is simple to fabricate, set up and use as well as easy to transport.

Thus, the present invention relates to an equipment for rehabilitative respiratory physiotherapy according to claim 1.

In particular, the equipment according to the present invention comprises a mask or mouthpiece, supplying means to supply air to the mask or mouthpiece, a pressure gauge arranged in fluidic connection with the mask or mouthpiece and means for producing pneumatic vibrations in the air supplied to the mask or mouthpiece.

The supplying means supply an air flow rate lower than 250 cm³/s, or lower than 15 liters/minute, at a pressure substantially equal to the atmospheric pressure. In other words, the equipment according to the present invention is not intended to compensate for a pulmonary failure of the patient, which evidently has to be self-sufficient in this regard.

The pressure gauge is designed to detect the depression the self-sufficient patient generates in the mask or mouthpiece when he/ she starts inhaling air. Basically, the pressure gauge detects the beginning of the patient breathing in. Automatically, the pressure gauge produces an activation signal for activating the means for producing pneumatic vibrations.

Unlike known solutions, the equipment according to the present invention is designed to intervene during the breathing in phase of the self-sufficient patient which does not need assisted pulmonary ventilation. The equipment produces one or more pneumatic vibrations in the breathed in air flow. Preferably, the frequency of the vibrations can be adjusted by the operator up to a determined time-constant value higher than 15 Hz, for example between 15 and 100 Hz, or else it can be adjusted continuously during the breathing in phase in a range of values always higher than 15 Hz, for example between 15 Hz and 100 Hz.

The pneumatic vibrations produced in the air supplied to the mask, or mouthpiece, have a minimum amplitude. For example, being the air flow rate during the breathing in phase of the patient about 15 liters per minute, the flow rate oscillations (positive or negative) caused by the vibrations produced by the equipment are always less than 6 liters per minute and mostly less than 1 liter per minute. Therefore, the vibrations cause minimal fluctuations of the flow rate value and, similarly, of the pressure value (the pressure pattern is qualitatively similar to the flow rate pattern). For this reason we can say that the vibrations generated by the equipment do not bring about pressure peaks potentially dangerous to the patient.

The vibrations produced in the breathed-in air flow are naturally transmitted to the inner walls of the airways of the patient, and in particular are transmitted to his lungs. Advantageously, the mechanical action of vibrations on the tissues easily removes the secretions covering the inner walls of the airways. Therefore, it is noticed a better ability to expectorate compared to what is possible to find with the use of known equipments, with the same treatment time.

Due to the fact that the equipment does not replace the normal activity of the patient's lung, and supplies an air flow rate corresponding to the volume normally breathed-in at rest, any risk of causing trauma to the lungs is prevented. Therefore, the equipment can be used directly by the patient, even at home, without the need of medical staff.

More clearly, the equipment according to the invention is intended to be used mostly in a domestic environment. The function of the air flow supplied to the mask or mouthpiece is not to insufflate air in the lungs of the patient, but only to convey the vibrations to be transmitted to walls of his/her airways to get the detachment of secretions.

In a first embodiment, the equipment according to the present invention comprises motorized supplying means to supply an air flow rate to the mask or mouthpiece. In this arrangement, the pressure gauge activates the supplying means at the beginning or during the breathing in phase of the normal respiratory cycle of the patient. The means for producing pneumatic vibrations in the breathed in air flow are constituted precisely by the supplying means.

For example, the supplying means comprise a compressor which inherently transmits pneumatic vibrations to the air flow delivered to the mask or mouthpiece, during operation.

In the preferred embodiment, the supplying motorized means can be adjusted to change, according to the needs, one or more characteristics of the supplied air flow, for example the flow rate, the amplitude and/or the frequency of the vibration or vibrations conveyed by the air, etc.

In the preferred embodiment, the equipment comprises a compressor, for example a positive-displacement compressor, using pistons, vanes, etc., driven by an electric motor. The compressor can produce, for example, air flows with flow rates ranging from 0.5 to 15 l/min, and pressures to the mask or mouthpiece that are negligible compared to atmospheric pressure. By adjusting the rotation frequency of the motor and, therefore, of the compressor, the desired frequency of the vibration or vibrations caused in the air flow delivered to the mouthpiece is achieved.

By adopting some known tactics, such as adopting air-relief or by-pass valves in the compressor, or else a mechanical reduction unit for connecting to the electric motor, etc., it is possible to achieve the desired adjustment of the supplied air flow rate.

Preferably, an exhaust opening of the air to the atmosphere is provided between the supplying means and the mask or mouthpiece, or directly on the mask or mouthpiece. In this case, it is clear that the pressure of the air flow rate supplied to the patient is substantially equal to the atmospheric pressure.

Preferably, the mask/mouthpiece is provided with a one-way valve to draw air directly from the environment. In this way, the patient breathes in an air flow rate through the one-way valve and simultaneously a vibrated air flow rate coming from the compressor.

During the breathing out phase the equipment can work according to two operating modes. In the first mode, the motor activating the compressor is turned off; in the second operating mode, the compressor keeps working but will vent into the atmosphere instead of delivering the air flow to the mask or mouthpiece. In this second mode, the pressure gauge also activates an air-relief valve on the compressor delivery to divert the flow into the atmosphere such that it does not reach the mask or mouthpiece.

Preferably, the compressor is provided with a valve to adjust the sucked air flow rate; by adjusting the valve, the instantaneous air flow rate the compressor can suck from outside and thus can deliver to the mask/mouthpiece, is correspondingly adjusted.

In a second embodiment of the invention alternative to the first one, the pneumatic vibrations are produced by one or more vibrating or oscillating membranes arranged in contact with the air flow that will pass through the mask/mouthpiece. The supplying means are constituted exactly by the vibrating membrane. For example, a membrane can be arranged as an audio speaker, i.e. activated by an electromagnetic coil in response to the activation signal generated by the pressure gauge.

In a third embodiment of the invention, a mechanical lung, i. e. a device designed to deliver the same air flow rate to the mask/mouthpiece and suck out the same air flow rate therefrom alternately, possibly without compression or with a minimum compression, is used to transmit vibrations to the air flow the patient breathes in.

For example, the mechanical lung can be made as a motorized bellows in which the delivery and the intake coincide and are both constituted by the duct connecting the bellows to the mask/mouthpiece.

In this third embodiment, the work done by the lung does not affect the respiratory cycle of the patient, but produces vibrations in the breathed in air flow with the a substantially sinusoidal pattern over time of slight overpressure.

In the different embodiments of the invention, the function of the pressure gauge, for example of a mechanical, electromechanical or electronic type, is to detect when the breathing in phase starts or, in other words, or detect the instantaneous depression that is generated in the mask/mouthpiece or in a volume fluidically connected to it when the patient -at the end of the breathing out phase- starts a new breathing in phase. The activation signal is preferably electric and activates the motor controlling the compressor, or activates an oscillating membrane, etc.

In general, when the beginning of the breathing in phase is detected, the pressure gauge activates the means for producing the pneumatic vibrations almost immediately, or with a time delay.

For example, in this second case, the equipment is provided with an electronic control circuit allowing the patient to adjust a time delay for the activation of the means for transmitting the vibrations, for example 0.5 seconds or 1 second after detecting the beginning of the breathing in phase. Due to this activation delay, the vibrations are transmitted to the air when the latter has already been partially accelerated by the patient breathing in.

In the various embodiments, preferably, the mask/mouthpiece is provided with a hand valve for adjusting the air flow rate the patient breathes out. This valve, as in PEP devices, acts to curb the breathed out air providing a resistance such as to prevent part of the airways from collapsing. The valve is of a ring nut type provided with calibrated holes, and is positioned on the mask/ mouthpiece.

The equipment can be provided with a tool prearranged to detect and show air pressures to the patient both in the breathing in and out phases, the pressures being affected by the position of the aforesaid ring nut.

Optionally, the equipment according to the present invention can be provided with means for the aerosol therapy which can be supplied by the forced air flow from the compressor, if present, to the mask/ mouthpiece.

Alternatively, the equipment can be complemented with a nebulizer (pneumatic or ultrasonic) directly coupled to the mask/ mouthpiece which has the function to nebulize a physiological solution adapted to humidify the respiratory system of the patient. If an ultrasonic nebulizer is used, the delivering temperature of the aerosol is between 25° and 30 ° C, to fluidify the secretion and limit the bronchospasms due to fresh air otherwise inhaled by the patient himself with standard pneumatic nebulizers.

The present disclosure, in a second aspect thereof, relates to a method for operating an equipment for rehabilitative respiratory physiotherapy. The method can be used with equipments provided with a mask or mouthpiece, a pressure gauge prearranged in fluid connection with the mask/mouthpiece and means for producing pneumatic vibrations in the air flow passing through the mask or mouthpiece, wherein the pressure gauge is arranged in logic connection with the means for producing the vibrations. The method provides the steps of:
a) detecting when the patient starts the breathing in phase, by means of the pressure gauge;
b) subordinately to the success of the detection in the step a), generating an activation signal for activating the means for producing pneumatic vibrations in the breathed in air flow.

It is possible to carry out step b) with a predetermined delay with respect to step a).

The frequency of the vibrations produced in the air flow can be adjusted preferably between 15 and 100 Hz.

### List of the figures

Further characteristics and advantages of the present invention will be more evident from a review of the following specification of a preferred, but not exclusive, embodiment, shown for illustration purposes only and without limitation, with the aid of the attached drawings, in which:
- figure 1 is a schematic side elevation view of a first equipment according to the present invention;
- figure 1A is a schematic view of an equipment according to the present invention;

- figure 2 is a schematic front elevation view of the first equipment;
- figure 3 is a schematic rear elevation view of the first equipment;
- figure 4 is a perspective view of a second equipment according to the present invention;
- figure 5 is a perspective schematic view of a third equipment according to the present invention;
- figure 5A is a partial sectional view in vertical section of the equipment shown in figure 5;
- figures 6 to 10 are diagrams relating to experimental tests carried out on the first equipment in corresponding conditions of use, showing the frequency of the vibrations produced in the air flow delivered to the mouthpiece;
- figure 11 is a diagram relating to the amplitude of the vibrations produced in the air flow delivered to the mask/mouthpiece;
- figure 12 is a time/flow rate diagram showing the usual respiration pattern of a patient;
- figure 13 is a time/flow rate diagram showing the respiration pattern of a patient using the equipment according to the present invention, calibrated to produce vibrations of maximum amplitude;
- figure 14 is a time/flow rate diagram showing the respiration pattern of a patient using the equipment according to the present invention, calibrated to produce minimum amplitude vibrations.

### Detailed description of the invention

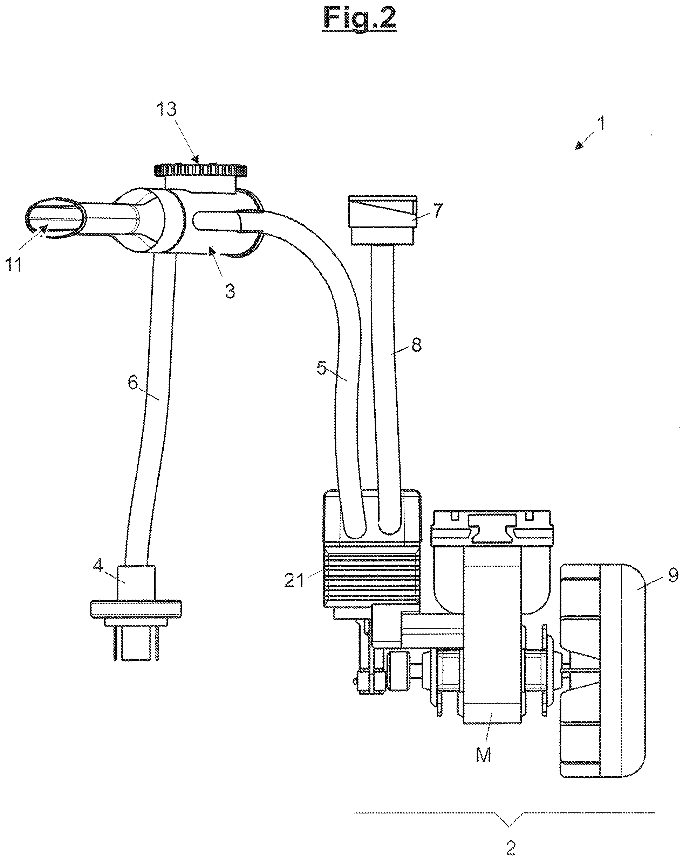

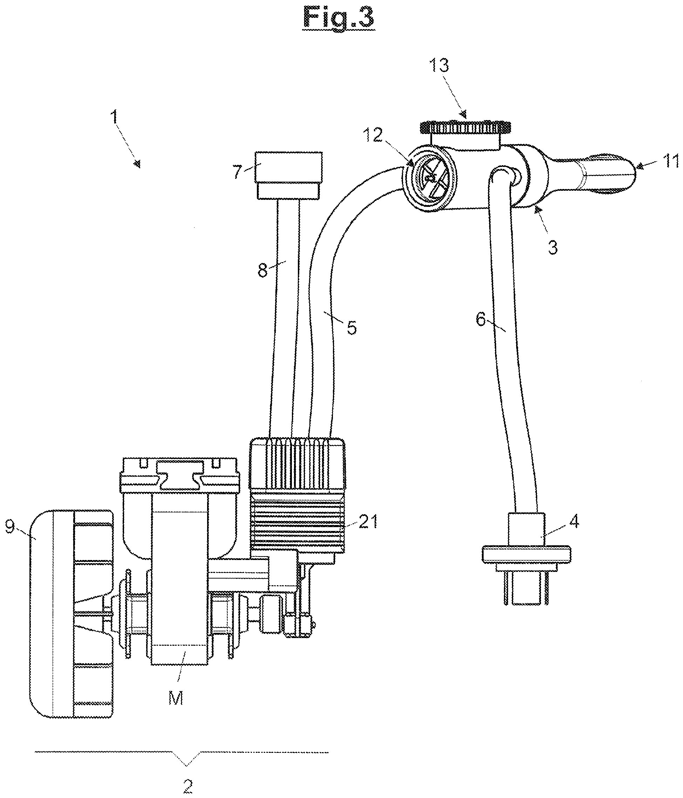

Referring to figures 1 to 3, the numeral **1** generally relates to an equipment according to a first embodiment of the present invention. The equipment **1** comprises supplying means **2** comprising in turn a rotary compressor **21** and the respective motor **M,** a mouthpiece **3** and a pressure gauge **4.**

The delivery of the compressor **21** supplies air to the mouthpiece **3** by means of the duct **5.** The pressure gauge **4** of the mechanical, or electrical, electromechanical, electronic, etc., type is connected to the inner volume of the mouthpiece **3** by means of the duct **6.** The pressure gauge **4** is also operatively connected to the motor M of the compressor **21,** for example by an electrical connection (not shown) or to a controller of the compressor **21.**

The compressor **21** draws an air flow rate from the environment through the duct 8 shut off by the adjustable valve 7. The operator can operate the shut-off valve 7 to change the air flow rate sucked by the compressor **21** and delivered to the mouthpiece **3.**

The air flow rate the compressor **21** supplies to the mouthpiece **3** is constant and lower than 15 liters per minute or 250 cm³ per second.

The numeral **9** indicates the possible cooling fan of the compressor **21.**

The pressure gauge 4 is calibrated to detect the beginning of each breathing-in phase of the patient during the physiotherapy session. Preferably, the sensitivity of the pressure gauge can be adjusted; this allows the equipment **1** to be used both with adults and children, indifferently. Substantially, the pressure gauge **4** detects the instantaneous depression caused in the mouthpiece **3** and in the duct **6** by the patient when he/she, by breathing in autonomously, sucks the air from the mouth **11** of the mouthpiece 3 itself.

Optionally, as schematically shown in figure 1A, the equipment **1** comprises a tool **10** that detects the instantaneous value of the air pressure in the mouthpiece **3** and shows it to the patient. The physiotherapist is then able to give the patients the parameters concerning the breathing in and out phases to allow the patient to accurately carry out the therapy without supervision.

The equipment **1** operates as follows.

The patient breathes out through the mouthpiece **3.** The breathed out air leaves the mouthpiece **3** through an adjustable ring nut valve **13** acting as a PEP device. If there is the tool **10,** it helps to understand the results of the adjustments made by the valve **13.**

At the end of the breathing out phase, the patient starts the breathing in phase while sucking out air through the mouth **11** of the mouthpiece **3.** In this way, a temporary and instantaneous depression is produced in the mouthpiece **3,** immediately detected by the pressure gauge **4** which produces an electric signal to activate the motor of the compressor **21.** The compressor **21** delivers an air flow rate to the mouthpiece **3** by means of the duct **5.**

In the first embodiment shown in figures, the mouthpiece **3** is further provided with a one-way valve **12** for the air entrance from outside. The air flow rate passing through the valve **12** adds to the air flow supplied by the compressor **21.**

As the compressor **21** rotates, it transmits to the air delivered to the mouthpiece **3** a pneumatic vibration having a frequency proportional to the rotation speed of the compressor **21** itself.

As above described, the vibrated air breathed in by the patient exerts a mechanical effect on the inner wall of the airways; the tissues, in turn, vibrate such as to facilitate the detachment of the accumulated mucus.

Preferably, the compressor **21** is provided with mechanical and/or electrical adjusting means to adjust the rotation speed. The adjusting means (not shown) are accessible by the operator to achieve the best adjustment of the equipment case by case, according to the needs of the patient.

The air supply from the compressor **21** generates, in the patient airways, a slight instantaneous overpressure with respect to the instantaneous pressure usually measurable in the same patient during the breathing in phase. The overpressure is minimal; what matters is that the air is vibrated.

When the patient ends the breathing in phase, and starts the subsequent breathing out phase, the pressure gauge detects the instantaneous pressure change in the mouthpiece **3** and generates a corresponding signal. In one embodiment, the signal controls the switch-off of the compressor **21;** in another embodiment the compressor **21** remains on and the signal controls the opening of a by-pass outlet (not shown) of the air toward the atmosphere.

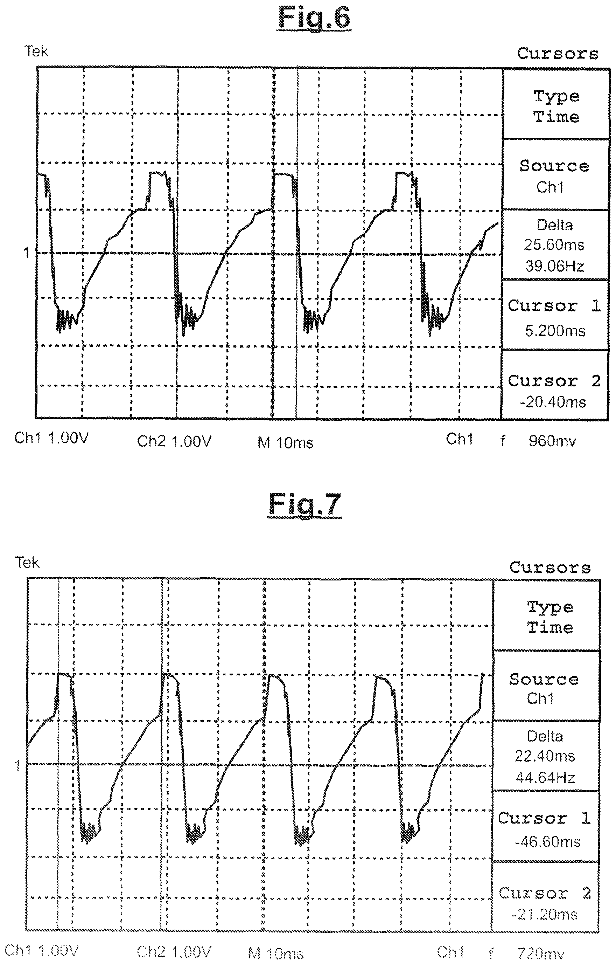

The characteristics of the air supplied to the mouthpiece **3** by the compressor **21** will be explained below with reference to figures 6-11.

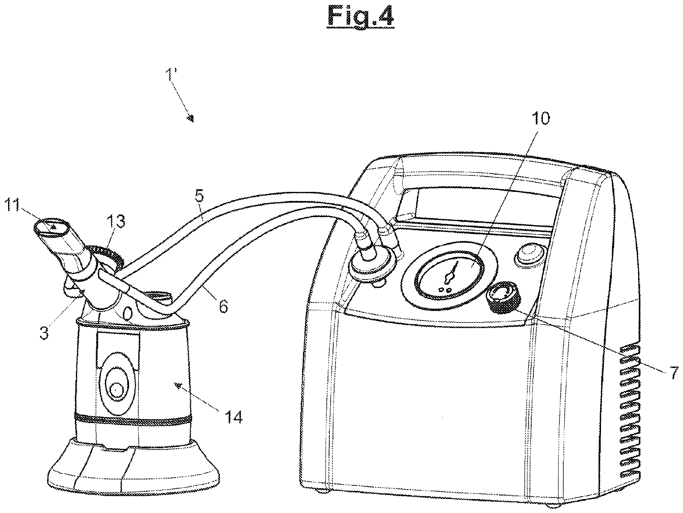

Figure 4 shows a second embodiment of the equipment **1'** according to the invention. The repeated reference numerals indicate components that are same or similar to those of figures 1-3. The compressor **21** and other components of the equipment **1'** are enclosed in a portable housing provided with a handle. In this embodiment, an accessory **14** consisting of a device for aerosol therapy is associated to the mouthpiece **3.** The compressor **21** supplies forced air which, if need be, is used to activate the device **14** and deliver to the mouthpiece **3** aerosolized drugs or physiological solution to hydrate the airways.

The device **14** is prearranged in series with the mouthpiece **3** and in parallel with the compressor **21.**

Figure 1A partially, and only schematically, shows a third embodiment of the equipment **1".** The reference numeral **15** indicates a device generating vibrations in the air flow supplied by the compressor **21** which, in this case, can be of any type. The device **15** is, for example, a vibrating membrane, or else an audio speaker arranged directly in contact with the air coming from the compressor **21** and delivered to the mouthpiece **3.** The frequency and amplitude of the vibrations of the speaker are adjustable at will, for example by means of a dedicated controller (not shown).

The equipments **1, 1', 1"** comprise, even if not shown, a controller to manage the various components, for example an interface or handles for the operator, which allow to independently adjust the air flow rate sucked out by the compressor **21** through the valve 7, the rotation speed of the motor M and/or of the compressor **21,** etc.

Preferably, to achieve the above described effects, the controller can be programmed to provide a time delay between the activation signal output by the pressure gauge **4** and the real activation of the compressor **21.**

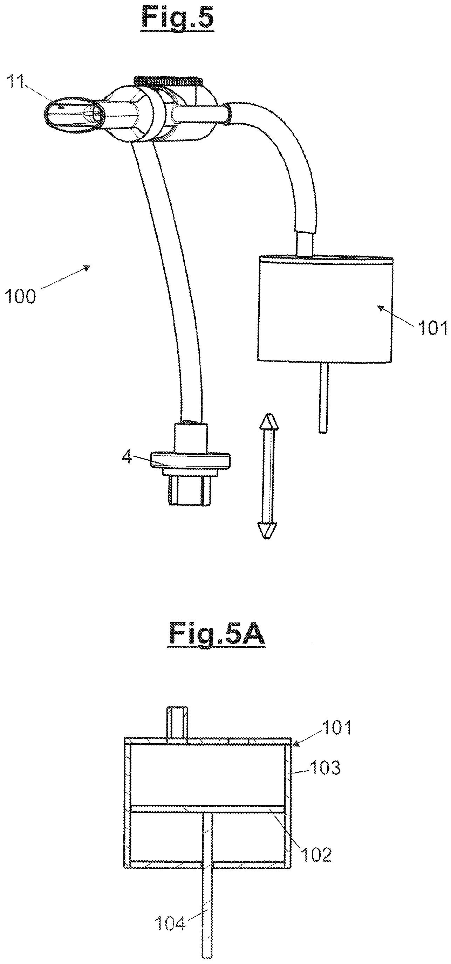

Figure 5A shows a third embodiment of an equipment **100** according to the invention. Alternatively or in addition to the compressor **21** or the vibrating membrane **15,** a mechanical lung **101** is provided fluidically connected to the duct **5.**

Figure 5A shows a schematic section of the mechanical lung **101,** substantially comprising a cylinder **103** in which a piston **102** is alternately pushed by an arm **104** translatable in the two directions shown by the arrow in figure 5.

Substantially, the lung **101** delivers an air flow rate to the mouthpiece 3 when the piston **102** goes up, and the same air flow rate is removed from the mouthpiece when the piston **102** goes down. Therefore, the mechanical lung **101** provides the air flow passing through the mouthpiece **3** with a corresponding pneumatic vibration almost sinusoidal.

The Applicant carried out lab tests for verifying the dynamics of vibrations transmitted to the air delivered to the patient. The tests were carried out according to the following procedures, which can also be used to contrast counterfeiting.

At the mouth **11** of the mouthpiece **3,** or inside it, a microphone is positioned. The audio signal detected by the microphone, when the compressor **21** is on and delivers air to the mouthpiece **3,** was analysed by an oscilloscope to identify the frequency and amplitude of the audio signal, clearly corresponding to the frequency and amplitude of the pneumatic vibrations in the air flow.

Figure 6 is a time-decibel diagram of the audio signal detected in a first use condition of the equipment **1,** the compressor **21** being on at a rotation frequency of about 40 Hz. In particular, decibels are expressed in millivolt. As it can be noted, the frequency of the oscillations detected by the microphone is about 39.06 Hz, i.e. it substantially corresponds to the rotation frequency of the compressor **21.** The amplitude of the oscillations mainly depends on the air flow rate supplied by the compressor **21,** which can be changed by the operator by acting on the valve 7.

Figure 7 is a diagram referred to a detected frequency of 44.64 Hz, that is a number of compressor revolutions higher than the case shown in figure 6.

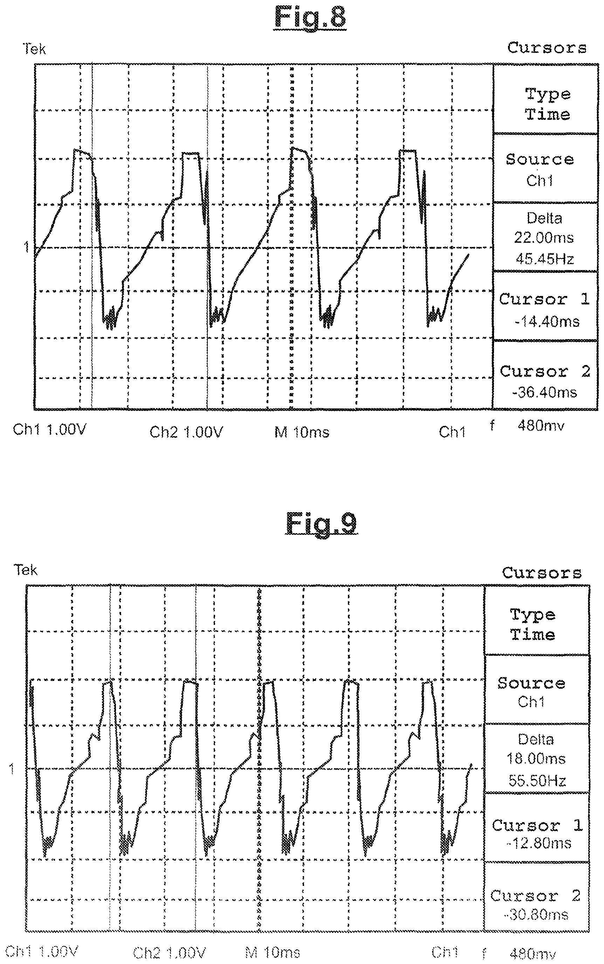

Figures 8, 9 and 10 respectively show the detected signal at a frequency of 45.45 Hz, 55.56 Hz and 83.33 Hz, obtained by increasing correspondingly the rotation speed of the compressor **21.**

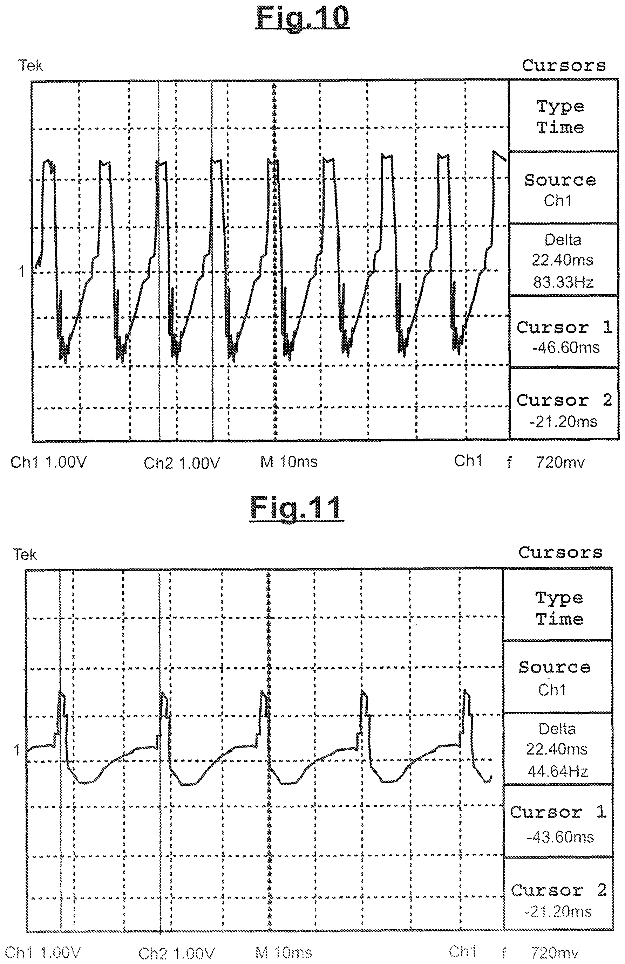

Figure 11 shows the diagram related to the pulse amplitude when frequency is 44.64 Hz and which denotes a flow rate of about 61/min, to be compared with the pulse amplitude in figure 6 where the flow rate is about 141/min.

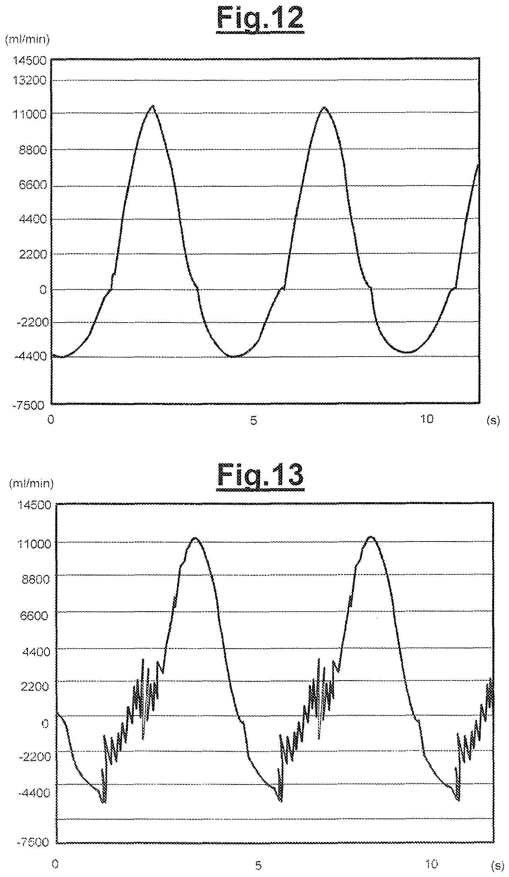

Figure 12 shows a diagram of the air flow rate (in millilitres per minute) a self sufficient patient normally breathes in and out. The diagram has been detected by applying a flowmeter in a mouthpiece through which a patient breathes, but not connecting it to the equipment according to the present invention.

Figure 13 shows the diagram of the air flow rate that a patient breathes in and out through the mouthpiece which is now coupled with the equipment according to the present invention, calibrated such as to generate maximum amplitude vibrations. As evident comparing figure 13 to figure 12, the equipment generates vibrations causing clear flow rate oscillations in the flow during the breathing in phase. The maximum amplitude of the oscillations is about 6 liters/min, but the oscillation amplitude has generally lower amplitude of about 1 liter/min.

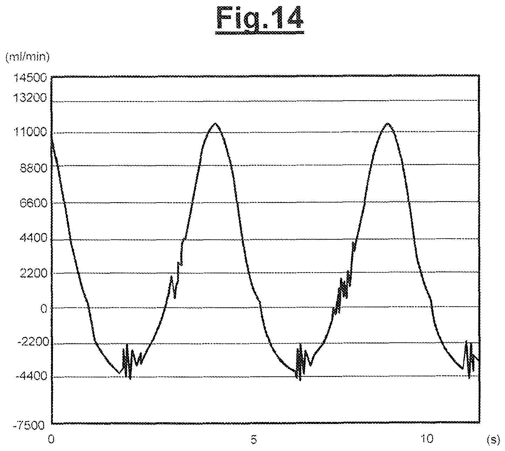

Figure 14 shows the diagram of the air flow rate that a patient breathes in and out through the mouthpiece, which is now coupled with the equipment according to the present invention, calibrated such as to generate minimum amplitude vibrations. As evident comparing figure 14 to figure 13, the equipment generates vibrations causing flow rate oscillations of lower amplitude in the flow during the breathing in phase.

The pilot study was carried out on three hyper-secretive patients of different age, sex and basic pathology.

### The three patients,

- F.M. male 74-year-old, hyper-secretive, chronic COPD in respiratory failure;
- S.R. male, 80-year-old, hyper-secretive, bronchiectatic and suffering from chronic myelogenous leukemia;
- S.C. female, 36-year-old, hyper-secretive, asthmatic,
performed a walking test on a flat surface for 6 minutes, generally called 6MWT (6 minutes walk test), before and after they underwent 30 minute treatment with the equipment **1** according to the present invention. The values of the arterial blood pressure ABP, the SaO₂ saturation and the heart rate HR were monitored.

In particular, a pilot clinical study to test the effectiveness of the device shown in figure 1 has been carried out.

The study results are as follows.

F.M. male 74-year-old showed a 10% increase of the 6MWT, the HR prior to treatment with the equipment 1 was 75 bpm and remained unchanged, SaO₂ increased from 80% to 89%, ABP from 130/60 mmHg to 110/60 mmHg.

S.R. male 80-year-old showed a 15% increase of the 6MWT, HR passed from 85 bpm to 84 bpm, SaO₂ from 95% to 100%, ABP from 120/80 mmHg to 110/75 mmHg.

S.C. female 36-year-old showed a 20% increase of the 6MWT, HR passed from 81 bpm to 69 bpm, SaO₂ from 95% to 100%, ABP from 90/60 mmHg to 85/60 mmHg.

**The table 1 below summarizes the results of the clinical pilot study.**

| TABLE 1 | | | | |
|---|---|---|---|---|
| | 6MWT | HR | SaO₂ | ABP |
| F.M. | > 10% | from 75 to 75 | from 80% to 89% | from 130/60 to 110/60 |
| S.R. | > 15% | from 85 to 84 | from 95% to 100% | from 120/80 to 110/75 |
| S.C. | > 20% | from 81 to 69 | from 95% to 100% | from 90/60 to 85/60 |

The use of the equipment **1** allowed to improve the respiratory parameters of the three patients, in terms of arterial oxygen saturation, endurance and exercise tolerance.

## Claims

1. Equipment (1) for rehabilitative respiratory physiotherapy, comprising a mask or mouthpiece (3), supplying means (2) to supply air to the mask or mouthpiece (3), a pressure gauge (4) arranged in fluidic connection with the mask or mouthpiece (3) and means (15) for producing pneumatic vibrations in the air supplied to the mask or mouthpiece (3), wherein the supplying means (2) supply an air flow rate lower than 250 cm³/s, or lower than 15 liters/min., at a pressure substantially equal to the atmospheric pressure, **characterized in that** the pressure gauge (4) is designed to detect the depression the patient generates in the mask or mouthpiece (3) when he/ she starts inhaling air and to generate a corresponding activation signal of the means for producing pneumatic vibrations.

2. Equipment (1) according to claim 1, wherein the frequency of said vibrations can be adjusted up to a determined time - constant value higher than 15 Hz, or else it can be adjusted continuously between several values higher than 15 Hz, for example between 15 Hz and 100 Hz.

3. Equipment (1) according to claim 1 or claim 2, wherein the supplying means (2) comprise a compressor (21) and the respective motor (M) and the vibrations are produced by the compressor (21) and transmitted directly to the supplied air to the mask or mouthpiece (3).

4. Equipment (1) according to claim 3, further comprising adjusting means to adjust the rotation speed of the compressor (21) and/ or the motor (M), said adjusting means being accessible by the operator to change the rotation speed of the compressor and/ the motor and at the same time to change the frequency of vibrations produced in the supplied air flow.

5. Equipment (1') according to any one of the preceding claims 1-2, wherein the means (15) for producing said vibrations comprise at least one vibrating membrane, prearranged in contact with the air flow supplied to the mask or mouthpiece (3).

6. Equipment (100) according to any one of the preceding claims 1-2, wherein the means for producing said vibrations comprise a mechanical lung (101) designed to deliver the same air flow rate to the mask or mouthpiece (3) and to suck out the same air flow rate therefrom alternately.

7. Equipment according to any one of the preceding claims 1-6, wherein the pressure gauge (4) is connected to the volume inside the mask or mouthpiece (3) by means of a duct (6) and it is electrically connected to the means for producing pneumatic vibrations.

8. Equipment according to any one of the preceding claims 1-4, wherein the supplying means (2) are designed to draw air from the surrounding environment and they further comprise a valve (7) for adjusting the drawable air flow rate.

9. Equipment according to any one of the preceding claims 1-8, wherein said mask or mouthpiece (3) comprises a first suction opening (12) for the ambient air, independently from the supplying means (2) or the means for producing pneumatic vibrations, and a second exhaust opening (13) for the breathed out air.

10. Equipment (1) according to claim 9, comprising a first on-off valve (12) for the air flow rate which can be sucked out through said first opening, and/ or a valve (13) for adjusting the air flow rate which can be breathed out through said second opening, and/ or an instrument (10) for detecting the air pressure in the mask or mouthpiece (3).

11. Equipment (1) according to claim 10, comprising a pneumatic or ultrasonic nebulizer (14), combined with the mask or mouthpiece (3) to add, to the flow the patient sucked out, potential drugs or physiological solution which is adapted to humidify the patient respiratory system.

12. Equipment (1) according to any one of the preceding claims 1-11, comprising a controller programmed to activate said means for producing pneumatic vibrations, in response to the activation signal generated by the pressure gauge (4), with a time delay adjustable by the operator.

13. Equipment (1) according to any one of the preceding claims 1-12, wherein the assembly formed by the supplying means, the mask or mouthpiece (3) and the line connecting the mask or mouthpiece (3) with the supplying means, defines a circuit open to the atmosphere, preferably next to at least one side opening of the mask or mouthpiece (3).

## Patentansprüche

1. Ausrüstung (1) für rehabilitative Atemphysiotherapie, die Folgendes umfasst:
eine Maske oder ein Mundstück (3), ein Zuführmittel (2) zum Zuführen von Luft zu der Maske oder dem Mundstück (3), ein Manometer (4), das in Strömungsverbindung mit der Maske oder dem Mundstück (3) angeordnet ist, und ein Mittel (15) zum Erzeugen pneumatischer Vibrationen in der Luft, die der Maske oder dem Mundstück (3) zugeführt werden, wobei das Zuführmittel (2) eine Luftströmungsrate von weniger als 250 cm³/s oder von weniger als 15 Litern/min bei einem Druck im Wesentlichen gleich dem atmosphärischen Druck zuführt, **dadurch gekennzeichnet, dass** das Manometer (4) dafür ausgelegt ist, die Eindellung zu detektieren, die der Patient in der Maske oder dem Mundstück (3) hervorruft, wenn er beginnt, Luft einzuatmen, und ein entsprechendes Aktivierungssignal des Mittels zum Erzeugen pneumatischer Vibrationen zu generieren.

2. Ausrüstung (1) nach Anspruch 1, wobei die Frequenz der Vibrationen bis zu einem bestimmten Zeitkonstantenwert eingestellt werden kann, der höher ist als 15 Hz, oder der ansonsten kontinuierlich zwischen mehreren Werten eingestellt werden kann, die höher als 15 Hz sind, zum Beispiel zwischen 15 Hz und 100 Hz.

3. Ausrüstung (1) nach Anspruch 1 oder Anspruch 2, wobei das Zuführmittel (2) einen Kompressor (21) und den jeweiligen Motor (M) umfasst und die Vibrationen durch den Kompressor (21) erzeugt und direkt zu der Luft übertragen werden, die zu der Maske oder dem Mundstück (3) geleitet wird.

4. Ausrüstung (1) nach Anspruch 3, die des Weiteren ein Einstellmittel zum Einstellen der Drehzahl des Kompressors (21) und/oder des Motors (M) umfasst, wobei der Bediener auf das Einstellmittel zugreifen kann, um die Drehzahl des Kompressors und/oder des Motors zu ändern und gleichzeitig die Frequenz der Vibrationen zu ändern, die in dem zugeführten Luftstrom erzeugt werden.

5. Ausrüstung (1') nach einem der vorangehenden Ansprüche 1-2, wobei das Mittel (15) zum Erzeugen der Vibrationen mindestens eine Vibrationsmembran umfasst, die zuvor in Kontakt mit dem Luftstrom angeordnet wird, welcher der Maske oder dem Mundstück (3) zugeführt wird.

6. Ausrüstung (100) nach einem der vorangehenden Ansprüche 1-2, wobei das Mittel zum Erzeugen der Vibrationen eine mechanische Lunge (101) umfasst, die dafür ausgelegt ist, im Wechsel die gleiche Luftströmungsrate zu der Maske oder dem Mundstück (3) zuzuführen und die gleiche Luftströmungsrate von dort abzusaugen.

7. Ausrüstung nach einem der vorangehenden Ansprüche 1-6, wobei das Manometer (4) mit dem Volumen im Inneren der Maske oder des Mundstücks (3) mittels eines Kanals (6) verbunden ist und elektrisch mit dem Mittel zum Erzeugen pneumatischer Vibrationen verbunden ist.

8. Ausrüstung nach einem der vorangehenden Ansprüche 1-4, wobei das Zuführmittel (2) dafür ausgelegt ist, Luft aus der Umgebung anzusaugen, und des Weiteren ein Ventil (7) zum Einstellen der Strömungsrate der ansaugbaren Luft umfasst.

9. Ausrüstung nach einem der vorangehenden Ansprüche 1-8, wobei die Maske oder das Mundstück (3) eine erste Absaugöffnung (12) für die Umgebungsluft, unabhängig von dem Zuführmittel (2) oder dem Mittel zum Erzeugen pneumatischer Vibrationen, und eine zweite Ausstoßöffiiung (13) für die ausgeatmete Luft umfasst.

10. Ausrüstung (1) nach Anspruch 9, die ein erstes Ein/Aus-Ventil (12) für die Luftströmungsrate, die durch die erste Öffnung herausgesaugt werden kann, und/oder ein Ventil (13) zum Einstellen der Luftströmungsrate, die durch die zweite Öffnung ausgeatmet werden kann, und/oder ein Instrument (10) zum Detektieren des Luftdrucks in der Maske oder dem Mundstück (3) umfasst.

11. Ausrüstung (1) nach Anspruch 10, die einen pneumatischen oder Ultraschall-Vernebeler (14) umfasst, der mit der Maske oder dem Mundstück (3) kombiniert ist, um der Strömung, die der Patient herausgesaugt hat, mögliche Medikamente oder eine mögliche physiologische Lösung, die geeignet sind, das Atemsystem des Patienten zu befeuchten, hinzuzufügen.

12. Ausrüstung (1) nach einem der vorangehenden Ansprüche 1-11, die einen Controller umfasst, der dafür programmiert ist, das Mittel zum Erzeugen pneumatischer Vibrationen in Reaktion auf das durch das Manometer (4) generierte Aktivierungssignal mit einer durch den Bediener einstellbaren Zeitverzögerung zu aktivieren.

13. Ausrüstung (1) nach einem der vorangehenden Ansprüche 1-12, wobei die Anordnung, die durch das Zuführmittel, die Maske oder das Mundstück (3) und die Leitung, welche die Maske oder das Mundstück (3) mit dem Zuführmittel verbindet, gebildet wird, einen Kreislauf, der zur Atmosphäre offen ist, bevorzugt neben mindestens einer seitlichen Öffnung der Maske oder des Mundstücks (3), definiert.

## Revendications

1. Équipement (1) pour la physiothérapie respiratoire de réhabilitation, comprenant un masque ou un embout buccal (3), des moyens d'alimentation (2) pour alimenter en air le masque ou l'embout buccal (3), un manomètre (4) disposé en connexion fluidique avec le masque ou l'embout buccal (3) et des moyens (15) de production de vibrations pneumatiques dans l'air alimentant le masque ou l'embout buccal (3), les moyens d'alimentation (2) fournissant un débit d'air inférieur à 250 cm³/s ou inférieur à 15 litres/min, à une pression substantiellement égale à la pression atmosphérique, **caractérisé en ce que** le manomètre (4) est conçu pour détecter la dépression que le patient génère dans le masque ou l'embout buccal (3) lorsqu'il/elle commence à inhaler air et à générer un signal d'activation correspondant pour les moyens de production de vibrations pneumatiques.

2. Équipement (1) selon la revendication 1, dans lequel la fréquence desdites vibrations peut être ajustée jusqu'à une valeur déterminée constante dans le temps supérieur à 15 Hz, ou bien elle peut être ajustée en continu entre plusieurs valeurs supérieures à 15 Hz, par exemple entre 15 Hz et 100 Hz.

3. Équipement (1) selon la revendication 1 ou la revendication 2, dans lequel les moyens d'alimentation (2) comprennent un compresseur (21) et le moteur respectif (M) et les vibrations sont produites par le compresseur (21) et transmises directement à l'air alimentant le masque ou l'embout buccal (3).

4. Équipement (1) selon la revendication 3, comprenant en outre des moyens d'ajustement pour ajuster la vitesse de rotation du compresseur (21) et/ou du moteur (M), lesdits moyens d'ajustement étant accessibles par l'opérateur pour modifier la vitesse de rotation du compresseur et/ou du moteur et en même temps pour changer la fréquence des vibrations produites dans le flux d'air alimenté.

5. Équipement (1') selon l'une quelconque des revendications précédentes 1-2, dans lequel les moyens (15) de production desdites vibrations comprennent au moins une membrane vibrante, pre-agencée en contact avec le flux d'air alimentant le masque ou l'embout buccal (3).

6. Équipement (100) selon l'une quelconque des revendications précédentes 1-2, dans lequel les moyens de production desdites vibrations comprennent un poumon mécanique (101) conçu pour fournir et aspirer, alternativement, le même débit d'air vers/de le masque ou l'embout buccal (3).

7. Équipement selon l'une quelconque des revendications précédentes 1-6, dans lequel le manomètre (4) est connecté au volume intérieur du masque ou de l'embout buccal (3) au moyen d'un conduit (6) et est connecté électriquement aux moyens de production de vibrations pneumatiques.

8. Équipement selon l'une quelconque des revendications précédentes 1-4, dans lequel les moyens d'alimentation (2) sont conçus pour aspirer l'air de l'environnement, et ils comprennent en outre une vanne (7) pour ajuster le débit d'air qui peut être aspiré.

9. Équipement selon l'une quelconque des revendications précédentes 1-8, dans lequel ledit masque ou embout buccal (3) comprend une première ouverture d'aspiration (12) pour l'air d'ambiance, indépendamment des moyens d'alimentation (2) ou des moyens de production de vibrations pneumatiques, et une seconde ouverture d'évacuation (13) pour l'air expiré.

10. Équipement (1) selon la revendication 9, comprenant une première vanne de sectionnement (12) pour le débit d'air qui peut être aspiré à travers ladite première ouverture, et/ou une vanne (13) pour ajuster le débit d'air qui peut être expiré à travers ladite deuxième ouverture, et/ou un instrument (10) pour détecter la pression de l'air dans le masque ou l'embout buccal (3).

11. Équipement (1) selon la revendication 10, comprenant un nébuliseur pneumatique ou ultrasonique (14) combiné avec le masque ou l'embout buccal (3) pour ajouter, au flux aspiré par le patient, des médicaments éventuels ou une solution physiologique qui est adaptée pour humidifier le système respiratoire du patient.

12. Équipement (1) selon l'une quelconque des revendications précédentes 1-11, comprenant un contrôleur programmé pour activer ledit moyen de production de vibrations pneumatiques, en réponse au signal d'activation généré par le manomètre (4), avec un retard temporel ajustable par l'opérateur.

13. Équipement (1) selon l'une quelconque des revendications précédentes 1-12, dans lequel l'ensemble formé par les moyens d'alimentation, le masque ou l'embout buccal (3) et la ligne connectant le masque ou l'embout buccal (3) aux moyens d'alimentation, définit un circuit ouvert à l'atmosphère, de préférence à côté au moins d'une ouverture latérale du masque ou de l'embout buccal (3).
